Europäisches Patentamt

European Patent Office

Office européen des brevets

(19)

(11) Numéro de publication: **0 371 850**
**A1**

(12) **DEMANDE DE BREVET EUROPEEN**

(21) Numéro de dépôt: 89403203.6

(22) Date de dépôt: 21.11.89

(51) Int. Cl.5: **A61B 19/04, A41D 19/00, A61F 6/04**

Le titre de l'invention a été modifié (Directives relatives à l'examen pratiqué à l'OEB, A-III, 7.3)

(30) Priorité: 01.12.88 FR 8815748

(43) Date de publication de la demande:
06.06.90 Bulletin 90/23

(84) Etats contractants désignés:
AT BE DE ES FR GB IT LU NL SE

(71) Demandeur: **HUTCHINSON**
**2 rue Balzac**
**F-75008 Paris(FR)**

(72) Inventeur: **Orlianges, Léo**
**4 ter Passage Legrand**
**F-92100 Boulogne(FR)**
Inventeur: **Argy, Gilles**
**15Ter Rue Nationale**
**F-78940 La Queue les Yvelines(FR)**
Inventeur: **Cheymol, André**
**15 Route de Descartes**
**F-86220 Dange-Saint-Romain(FR)**

(74) Mandataire: **Orès, Bernard et al**
**Cabinet ORES 6, Avenue de Messine**
**F-75008 Paris(FR)**

(54) **Gant, doigtier et analogue et procédés de fabrication.**

(57) La présente invention est relative à un gant, doigtier ou analogue caractérisé en ce que le latex dont il est constitué contient des microcapsules, microsphères ou microbilles (3, 4) dont la concentration croit à mesure que l'on s'éloigne de la surface extérieure du gant (1), concentration = 0, vers la surface intérieure du gant (2), au contact avec la peau de l'utilisateur, concentration = 90 à 95 % en microcapsules ou microbilles.

Applications : gant pour médecins, chirurgiens, personnels hospitaliers, etc...

FIG.1

## PERFECTIONNEMENTS APPORTES AUX GANTS, DOIGTIERS ET ANALOGUES DE PROTECTION ET DE MANIPULATION ET A LEURS PROCEDES DE FABRICATION

La présente invention est relative à des perfectionnements apportés aux gants ou doigtiers de protection et de manipulation notamment pour médecins, chirurgiens, personnels hospitaliers, chercheurs et techniciens.

Le type de gants le plus largement utilisé actuellement est le gant en latex facilement stérilisable et d'une asepsie parfaite, souple, résistant, adaptable à la morphologie de la main et d'une grande sensibilité tactile.

Toutefois ces gants, doigtiers ou analogues présentent deux gros inconvénients :
- des difficultés d'intromission et d'enlèvement
- des risques de déchirure par le scalpel ou de perforation par l'aiguille de la seringue.

Pour pallier la difficulté de l'intromission et faciliter le "glissement" on a eu recours à l'utilisation de différents additifs comme le talc ou l'amidon de maïs ou encore à la transformation chimique de la surface de contact avec la main par action d' halogènes. Ces additifs ou l'action d'halogènes (opération dite de "chlorination"), s'ils permettent en effet un meilleur glissement et un enfilage plus facile, provoquent souvent des réactions allergiques, qui dans certains cas peuvent être graves.

On a également proposé des doublures ou supports textiles, mais la solution est coûteuse et les gants obtenus sont d'un maniement difficile.

Quant aux risques de contamination que présentent la coupure ou la déchirure d'un gant, il n'y a pratiquement rien de prévu sur le marché actuellement.

La présente invention s'est en conséquence donné pour but de pourvoir à un gant, doigtier ou analogue de protection et de manipulation, qui répond mieux aux nécessités de la pratique que les gants ou les doigtiers précedemment proposés conformément à l'Art Antérieur, notamment en ce qu'il présente des propriétés de glissement subtantiellement améliorées sans avoir recours à différents additifs, ou des techniques qui soient sources de réactions allergiques, tout en assurant à l'utilisateur une protection accrue contre les germes ou autres agents pathogènes ou contre, par exemple, les piqûres dues aux aiguilles de la seringue.

La présente invention a pour objet un gant, doigtier ou analogue de protection et de manipulation caractérisé en ce que le latex ou analogue dont il est constitué contient des microcapsules, microsphères ou microbilles dont la concentration croît à mesure que l'on s'éloigne de la surface extérieure du gant, concentration = 0, vers la surface intérieure du gant, au contact avec la peau de l'utilisateur, concentration = 90 à 95 % en microcapsules ou microbilles.

La concentration élevée de ces microcapsules ou microbilles dans la partie du latex au contact avec la peau assure par le relief qu'elles donnent un glissement parfait et permet un enfilage très aisé des gants ne nécessitant donc aucun additif.

Selon un mode de réalisation particulièrement avantageux de l'objet de l'invention, les microcapsules ou microsphères dont le diamètre varie entre 10 et 100 microns, contiennent un agent pharmacologique approprié qui se répand sur la peau de l'utilisateur dès que l'enveloppe de la microsphère ou de la microcapsule est percée

La présence de ces microbilles qui donnent une surface de contact ondulée permet de diminuer les points de contact avec la peau de l'utilisateur et de réduire les forces de frottement ou l'adhérence entre la peau et le gant rendant ainsi aisée la mise en place du gant sur la main.

Toute percée du gant par l'aiguille de la seringue ou toute déchirure due à la manipulation du scalpel provoque ainsi automatiquement l'étalement de l'agent pharmacologique de protection à la fois sur l'aiguille ou la lame de scalpel et sur la partie de la peau atteinte. On peut régler la taille, l'épaisseur et la fragilité de l'enveloppe des microsphères ou des microcapsules de manière à ce que l'agent pharmacologique prophylactique se libère préalablement et plus ou moins facilement, par simple frottement, si on le désire.

Suivant une modalité particulière de ce mode de réalisation, les microsphères ou les microcapsules peuvent contenir différents agents pharmacologiques ayant des propriétés différentes et complémentaires.

Selon un autre mode de réalisation avantageux de l'objet de l'invention, la couche de latex ou analogue contient une substance deshydratante, telle qu'une résine acrylique par exemple. L'intérêt de ce mode de réalisation est évident pour un gant médical dont le port provoque souvent une transpiration. En présence de ladite substance déshydratante, la couche de latex ou analogue comprend préférentiellement des microbilles ou des microsphères ou microcapsules contenant un agent pharmacologique dépourvu d'eau.

Selon un autre mode de réalisation particulièrement avantageux de l'objet de la présente invention, la couche de latex ou analogue contient, dans sa partie dépourvue de microsphères ou microbilles (partie où le gradient de concentration en microsphères, microcapsules ou microbilles est nul), des barrières mécaniques contre les piqûres d'aiguille ou des coups de scalpel. Ces barrières mécaniques peuvent, avantageusement, par exemple, être constituées de microdisques de verre ou de fibres de bambou ou encore de fibres de carbone.

La présente invention a également pour objet un procédé de fabrication du gant ou doigtier de protection tel que défini plus haut, lequel procédé comporte les étapes suivantes :
- on réalise un premier bain pour façonner à la forme voulue la partie de la couche de latex ou de maté riau élastomère analogue dépourvue de microsphères, microcapsules ou microbilles, puis
- un deuxième bain dans un latex contenant la quantité voulue de microsphères, microcapsules ou microbilles, ou de substance déshydratante, l'epaisseur de cette partie étant fonction de la durée d'immersion,
- puis après la vulcanisation on procède au dépouillement du gant, doigtier ou analogue du moule et on le retourne.

Si on le désire, on peut faire précéder le premier bain d'un bain de coagulation et on peut précéder le deuxième bain d'une vulcanisation.

Suivant une modalité de l'invention, on peut déposer les microcapsules, microsphères ou les microbilles par flocage ou projection sur le film de latex avant le séchage.

Outre les dispositions qui précédent, l'invention comprend encore d'autres dispositions, qui ressortiront de la description qui va suivre, faite à titre d'exemple non limitatif et qui se réfère au dessin annexé dans lequel :
- la figure 1 représente en coupe à grande échelle, un exemple de réalisation d'une couche de latex conforme à la présente invention.
- la figure 2 représente une partie très grossie de la surface intérieure du gant et
- la figure 3 représente un autre exemple de réalisation d'une couche de latex conforme à la présente invention.

Il doit être bien entendu, toutefois, que ce dessin et les parties descriptives correspondantes sont donnés uniquement à titre d'illustration de l'objet de l'invention, dont ils ne constituent en aucune manière une limitation.

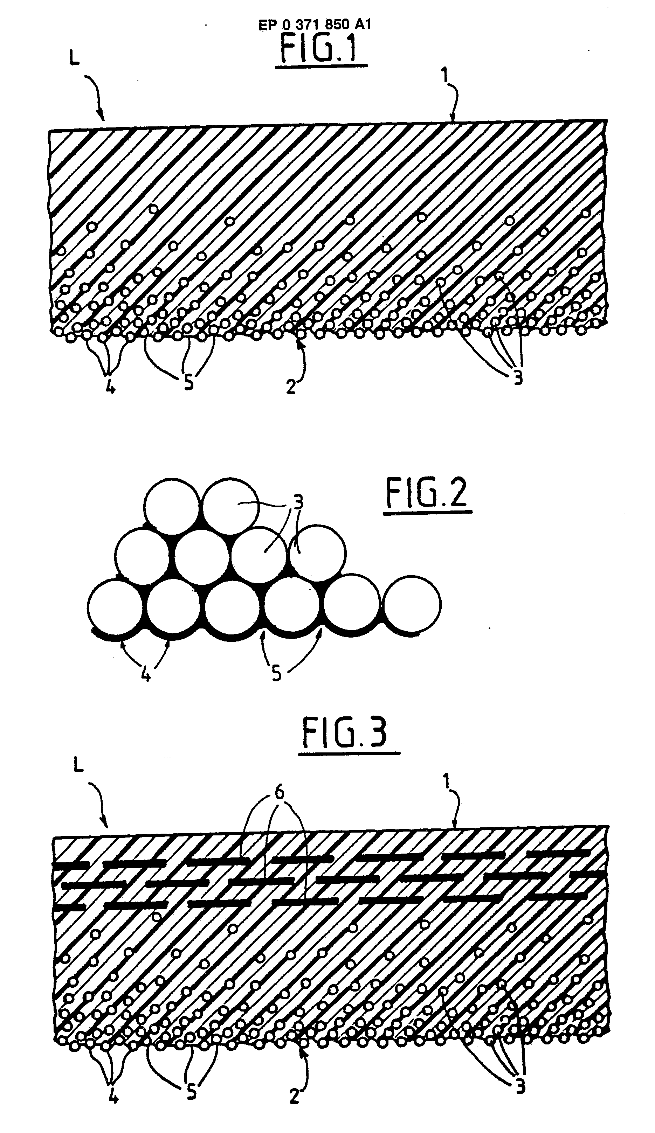

La préparation de la couche de latex L telle que représentée sur la figure 1 annexée où 1 représente la surface extérieure du latex, c'est-à-dire la surface exté rieure du gant, doigtier ou analogue et 2 représente la surface intérieure, appliquée contre la peau de l'utilisateur, laquelle surface intérieure est faite de creux 5 et de bosses 4, 3 désignant les microgranules, (que l'on distingue mieux sur la figure 2) a été réalisée de la manière suivante :
- on dépose sur une forme en céramique par immersion dans un latex une partie de la couche du doigtier puis on immerge après un léger séchage, dans un bain formé de latex prévulcanisé (par exemple "Resultex" prévulcanisé au soufre) dilué pour avoir une solution à 35 % d'extrait sec et dans lequel on a introduit les microgélules. L'immersion dure 15 secondes pour obtenir un film de 180 microns d'épaisseur. On rince pendant 10 minutes dans l'eau à 40° C, puis on sèche à température ambiante pendant 2 heures.

Les microsphères 3 sont préalablement remplies de l'agent pharmacologique désiré.

Une autre variante très intéressante de ce procédé consiste en l'introduction de microdisques 6 en verre (ou fibres de bambou ou fibres de carbone), comme représenté, par exemple, sur la figure 3.

Il résulte de la description qui précède que, quels que soient les modes de mises en oeuvre, de réalisation et d'application adoptés, l'on obtient des gants de protection qui glissent parfaitement grâce notamment à la présence des bosses 4 et des creux 5 et qui contiennent un agent pharmacologique renfermé dans les microsphères 3, qui présentent par rapport aux gants de protection visant aux mêmes buts antérieurement connus, des avantages importants, dont certains ont été mentionnés dans ce qui précède, et dont d'autres avantages ressortiront de l'utilisation desdits gants, doigtiers ou analogues.

## Revendications

1) Gant, doigtier ou analogue de protection et de manipulation caractérisé en ce que le latex ou le produit élastomère analogue dont il est constitué contient des microcapsules, microsphères ou microbilles dont la concentration croît à mesure que l'on s'éloigne de la surface extérieure du gant, concentration = 0, vers la surface intérieure du gant, au contact avec la peau de l'utilisateur concentration = 90 à 95 % en microcapsules ou microbilles.

2) Gant, selon la revendication 1, caractérisé en ce que les microcapsules, les microsphères ou les microbilles dont le diamètre varie entre 10 et 100 microns constituent une surface de contact ondulée diminuant notablement les forces de frottement entre la peau de l'utilisateur et le gant lors des opérations d'enfilage et d'enlèvement.

3) Gant selon la revendication 1 ou 2 caractérisé en ce que les microcapsules ou microsphères contiennent un agent pharmacologique approprié qui se répand sur la peau de l'utilisateur dès que l'enveloppe de la microsphère ou de la microcapsule est percée.

4) Gant selon la revendication 3 caractérisé en ce que les microcapsules ou les microsphères contiennent différents agents pharmacologiques.

5) Gant ou analogue selon la revendication 1 caractérisé en ce que la couche de latex ou matériau élastomère analogue contient une substance déshydratante absorbant l'eau ambiante et la sueur de la main.

6) Gant ou analogue selon la revendication 5 caractérisé en ce que le produit déshydratant est constitué par de la résine acrylique.

7) Gant, doigtier ou analogue selon la revendication 1 caractérisé en ce que le latex ou matériau élastomère analogue contient dans sa partie dépourvue de microsphères ou microbilles (partie où la concentration en mi crosphères, microcapsules ou microbilles est nulle), des barrières mécaniques contre les piqûres d'aiguille ou des coups de scalpel.

8) Gant ou analogue selon la revendication 6 caracterise en ce que lesdites barrières sont constituées par un matériau pris dans le groupe qui comprend des microdisques de verre, des fibres de bambou ou des fibres de carbone.

9) Procédé de fabrication des gants, doigtiers ou analogues selon l'une quelconques des revendications 1 à 8 caractérisé en ce que :
- on réalise un premier bain pour façonner à la forme voulue la partie de la couche de latex ou de matériau élastomère analogue dépourvue de microsphères, microcapsules ou microbilles, puis
- un deuxième bain dans un latex contenant la quantité voulue de microsphères, microcapsules ou microbilles, l'épaisseur de cette partie étant fonction de la durée d'immersion,
- puis après la vulcanisation on procède au dépouillement du gant, doigtier ou analogue du moule et on le retourne.

10) Procédé selon la revendication 9 caractérisé en ce que l'on précède le premier bain d'un bain de coagulation et le deuxième bain d'une vulcanisation.

11) Procédé selon la revendication 9 caractérisé en ce que les microcapsules, microsphères ou les microbilles sont déposées par flocage ou projection.

# FIG.1

L 1

4 5 2 3

# FIG.2

3

4 5

# FIG.3

L 6 1

4 5 2 3

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 771 482 (SHLENKER)<br>* Colonne 3, lignes 16-24,55-60; figure 6 *<br>--- | 1,3,4 | A 61 B 19/04<br>A 41 D 19/00<br>A 61 F 6/04 |
| A | US-A-4 779 290 (WELCH et al.)<br>* Colonne 2, ligne 61 - colonne 3, ligne 4 *<br>--- | 7,8 | |
| A | US-A-4 143 109 (STOCKUM)<br>* Colonne 3, lignes 4-22; ligne 31-64; figures 1,2,4 *<br>----- | 2,9 | |

DOMAINES TECHNIQUES
RECHERCHES (Int. Cl.5)

A 61 B
A 61 F
A 41 D
B 29 D
A 43 B
C 08 J

Le présent rapport a été établi pour toutes les revendications

| Lieu de la recherche | Date d'achèvement de la recherche | Examinateur |
|---|---|---|
| LA HAYE | 05-01-1990 | MOERS R.J. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
............................................................................
& : membre de la même famille, document correspondant

EPO FORM 1503 03.82 (P0402)